Europäisches Patentamt

European Patent Office

Office européen des brevets

(11) Publication number: **0 669 393 A1**

## EUROPEAN PATENT APPLICATION
### published in accordance with Art. 158(3) EPC

(21) Application number: 94926367.7

(22) Date of filing: **07.09.94**

(86) International application number:
**PCT/JP94/01476**

(87) International publication number:
**WO 95/07343 (16.03.95 95/12)**

(51) Int. Cl.⁶: **C12C 11/09**, C12G 1/073, C12G 3/02

(30) Priority: **07.09.93 JP 246128/93**
**27.12.93 JP 347067/93**

(43) Date of publication of application:
**30.08.95 Bulletin 95/35**

(84) Designated Contracting States:
**BE DE DK FR GB NL**

(71) Applicant: **SAPPORO BREWERIES LTD.**
**20-1, Ebisu 4-chome,**
**Shibuya-ku**
**Tokyo 150 (JP)**

(72) Inventor: **SHINDO, Masashi Sapporo**
**Breweries Limited Brewing**
**Research Laboratories**
**10, Okatohme**
**Yaizu-shi**
**Shizuoka-ken 425 (JP)**

(74) Representative: **VOSSIUS & PARTNER**
**P.O. Box 86 07 67**
**D-81634 München (DE)**

(54) METHOD OF PRODUCING LIOUOR.

(57) A method of producing a liquor, comprising the steps of feeding yeast to be fixed into a fluidized bed reactor equipped with a liquid circulation pipe and a gas exhaust port, supplying a brewing raw material liquid to the reactor, extracting a part of a culture liquid from the top of the reactor and returning the culture liquid into the bottom of the reactor. By this method fermentation is conducted while establishing circulation. Thus, a fluidized bed reactor filled with fixed yeast is used, fermented liquor in the reactor is circulated so as to move the fixed yeast. Accordingly, most of the carbon dioxide produced can be discharged outside the system without being dissolved in the fermented liquor. Moreover, amino acids in the brewing raw material liquid can be efficiently utilized by the yeast, so that the amino acid concentration in the product is low and the flavor is excellent.

Rank Xerox (UK) Business Services
(3.10/3.09/3.3.4)

EP 0 669 393 A1

# FIG.1

Field of Technology

The present invention relates to a process for producing a liquor. More specifically, the present invention relates to a process for producing a liquor which facilitates exhaust of carbon dioxide gas formed during fermentation, which expedites assimilation of amino acids in a raw material by yeast, and which produces the liquor having a stable flavor rapidly.

Background Technology

In the procedure of producing liquors such as beer, wine and the like, carbon and nitrogen sources in a raw material for brewing are generally consumed by yeast to form ethyl alcohol.

Since a technology of immobilizing yeast cells was developed such as a technology of immobilizing yeast by including the yeast in a hydrous gel, a process for producing beer by continuous brewing of a wort has been proposed (J. Inst. Brew., 84, 228 (1981)).

Since the yeast cells can be used in a high concentration in this process, a brewing period can be greatly reduced. However, the production of liquors such as beer and the like by packing the immobilized yeast cells into a packed bed-type reactor involves the following problems because of carbon dioxide gas formed during the fermentation.

(1) The dissolved carbon dioxide gas formed at a high level influences metabolic activity of the yeast.

(2) Accumulation of the carbon dioxide gas gives a dead space to reduce a solid-liquid contact area.

(3) Gel particles are deformed due to a pressure exerted by the carbon dioxide gas to promote close adhesion of the particles each other, thereby clogging a passage of the gas or the solution to give a nonhomogeneous flow.

In order to solve such problems, a method in which the carbon dioxide gas formed within the packed bed-type reactor is pressurized to dissolve it in the solution has been attempted (EBC Congress, Proc., 505 (1981)). However, the influence on the metabolic activity of the yeast is not eliminated by this method.

By the way, a bioreactor which is used in the fermentation is roughly classified into three types: (1) a stirred tank-type reactor, (2) a packed bed-type reactor and (3) a fluidized bed-type reactor. These reactors have their own merits and demerits (Reasonable Designing and Optimum Operation of Bioreactor, 151 (1986) published by Technical Information Center).

Of these reactors, the stirred tank-type reactor and the fluidized bed-type reactor bring forth the high effect to exhaust the carbon dioxide gas. Especially, the fluidized bed-type reactor has characteristics that the temperature and pH can be easily controlled, moving characteristics of substances are good and the pressure loss of the immobilized yeast cells is small. Accordingly, the fluidized bed-type reactor has been used in the fermentation of alcohol.

In the alcohol fermentation using the fluidized bed-type reactor, a method in which an inert gas is introduced from a lower portion of the reactor has been proposed to cause fluidizing of the immobilized yeast cells and to exhaust the carbon dioxide gas well (Chem. Eng. Sci., 19, 215 (1964)). However, this method incurs a high cost in recovery of the gas which is, therefore, not practical. Further, a method using mechanical stirring involves a fatal defect that the immobilized yeast is destroyed. Still further, when the packed bed-type reactor is packed with the immobilized yeast to conduct the fermentation, a degree of assimilation of amino acids in a raw material by yeast is low, and the concentrations of the amino acids in the fermentation solution remained high which causes a problem of a flavor.

It is an object of the present invention to provide a process for producing a liquor efficiently with the use of immobilized yeast cells upon solving the above-mentioned problems.

Disclosure of the Invention

The present invention firstly relates to a process for producing a liquor which comprises loading immobilized yeast cells into a fluidized bed-type reactor fitted with a liquid circulation pipe and a gas exhaust port, feeding a raw material for brewing to the reactor, withdrawing part of the culture liquid from the upper portion of the reactor, and returning the culture liquid to the inside of the reactor from the lower portion of the reactor to thereby conduct the cultivation while making a liquid circulation.

The present invention secondly relates to a process for producing a liquor wherein during the above-mentioned cultivation, the raw material for brewing is fed to the reactor, and part of the culture liquid is withdrawn outside the reactor to produce the liquor continuously.

The present invention employs the method to cause the immobilized yeast cells to fluidize by circulating the liquid instead of the above-mentioned method to introduce the inert gas from the lower portion of the reactor.

Brief Description of Drawings

Fig. 1 is a view illustrating an example of a fluidized bed-type reactor which is used in the present invention.

Fig. 2 is a graph showing a change with time of an apparent extract during the continuous fermentation in Example 1.

In the drawings, (1) is a wort tank, (2) is a pump, (3) is a pH sensor, (4) is a fluidized bed-type reactor, (5) is a temperature sensor, (6) is a gas exhaust port, and (7) is a product tank.

Best Mode to practice the Invention

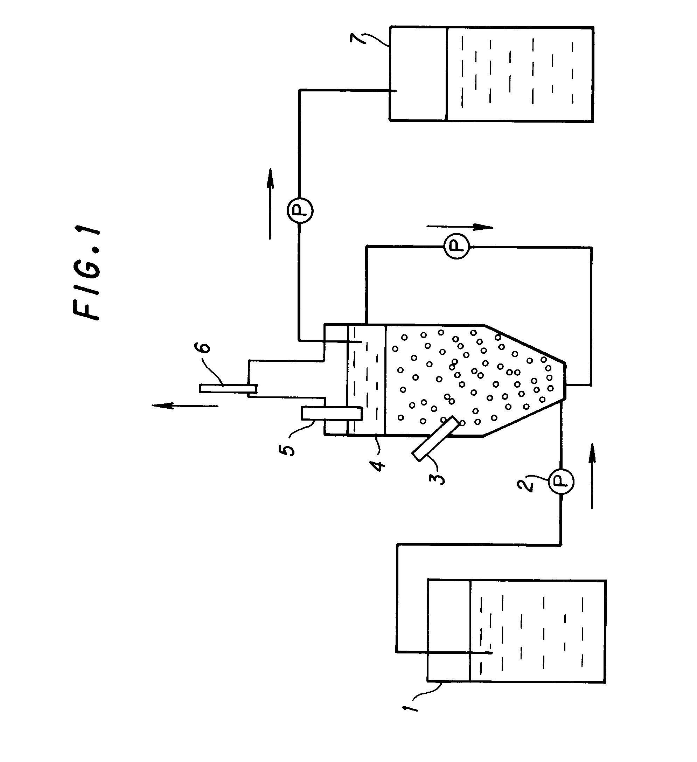

In the present invention, a liquor is produced by means of the fluidised bed-type reactor shown in Fig. 1. This reactor facilitates the exhaust of formed carbon dioxide gas by forcibly circulating part of the fermentation liquid. Specifically, as shown, the fermentation liquid in the reactor is withdrawn from the upper portion of the reactor and introduced into the lower portion via the pipe to allow the fermentation liquid to fluidize. Accordingly, the carbon dioxide gas formed during the fermentation is exhausted outside the reactor via the gas exhaust port without being dissolved in the liquid. This can avoid the undesirable influence of the carbon dioxide gas on the metabolic activity of yeast.

The raw material for brewing is any material which is suitable for proliferation of liquor producing yeasts such as beer yeast, wine yeast, sake yeast and the like. Any known material can be used arbitrary. Ordinarily, a wort, a fruit juice, a sugar solution, a saccharified grain solution and the like can be used either singly or in combination. Moreover, a suitable nutrient can be added as required.

The raw material for brewing is sterilized in a usual manner and then fed to the fluidized bed-type reactor loaded with the immobilized yeast. The feed amount of the material is from 0.05 to 0.2 $hr^{-1}$, preferably from 0.1 to 0.2 $hr^{-1}$ in terms of space velocity. The space velocity here referred to is calculated by multiplying an amount, per unit time, of the raw material being fed to the fluidized bed-type reactor by the amount of the immobilization support material having the yeast cells supported thereon. A suitable amount of the immobilization support material having the yeast cells supported thereon is from 5 to 50 % by volume, preferably from 10 to 30 % by volume based on the volume of the reactor.

The raw material can be introduced from a suitable position such as an upper portion, a lower portion or the like of the reactor.

As the yeast used to produce the liquor, a so-called liquor producing yeast that forms an alcohol, carbon dioxide gas or the like by metabolizing the raw material is used. Examples of the yeast include _Saccharomyces cerevisiae_, and _Saccharomyces uvarum_. Liquor producing yeasts such as beer yeast, wine yeast, sake yeast and the like may be selected appropriately according to purposes for use and the like. Examples thereof include _Saccharomyces cerevisiae_ OC-2 (IAM 4175). _Saccharomyces cerevisiae_ Kyokai wine yeast No. 1, _Saccharomyces cerevisiae_ Kyokai wine yeast No. 3, and _Saccharomyces cerevisiae_ Kyokai wine yeast No. 4. These yeasts are generally well known. The first strain has been preserved in Institute of Applied Microbiology in The University of Tokyo, and the other three strains in Foundation of Japan Brewing Association, respectively. Therefore, these yeasts can be obtained easily by third person.

These yeasts are generally facultative anaerobic. The support material (or carrier) for supporting the yeast cells includes various materials. Chitosan beads, alginic acid beads, and carrageenan beads are especially preferable. Ceramic beads and glass beads are not suitable as the support material of the present invention because they are less abrasion-resistant. The immobilization of the yeast is known per se (see, for example, Enzymology, compiled by Fukui Saburo, Chibata Ichiro and Suzuki Shuichi (published by Tokyo Kagaku Dojin), and David Williams, Douglas M. Munnecke, Biotec. and Bioeng., 23, 1813 (1981)). In the present invention as well, the immobilization may be conducted in the known manner.

The fermentation conditions may be basically known fermentation conditions. The fermentation temperature is usually 15°C or below, preferably from 8 to 10°C in the brewing of beer, and is usually approximately 20°C, preferably from 15 to 20°C in the brewing of wine.

The circulation rate of the fermentation liquid which is circulated during the fermentation is from 150 to 400 ml per minute, preferably from 200 to 250 ml per minute in, for example, a 1-liter reactor. When the fermentation is conducted under such conditions, the fermentation liquid is almost in a completely mixed state. Thus, the fermentation can be performed at good efficiency.

Since the fluidized bed-type reactor used in the present invention is good in the moving characteristics of substances as noted above, amino acids and the like contained in the raw material are taken into the yeast during the fermentation and assimilated by the yeast. Consequently, the concentrations of the amino acids in the liquor product are reduced in comparison to the product by the ordinary method, providing a good flavor.

The process of the present invention can be applied to the production of beer as well as other liquors such as wine, sake and the like.

Examples

The present invention is illustrated more specifically by referring to the following Examples.

Example 1

A 1-liter fluidized bed-type reactor was loaded with 250 ml of a support material (chitosan beads) having beer yeast [Saccharomyces cerevisiae IAM 4206 (ATCC 9080)] immobilized thereon, and 750 ml of a wort in which original extract of the wort was adjusted to 11 % Plato. The batchwise fermentation operation was conducted at 11°C upon setting a circulation rate of a fermentation liquid in the reactor at 250 ml/min.

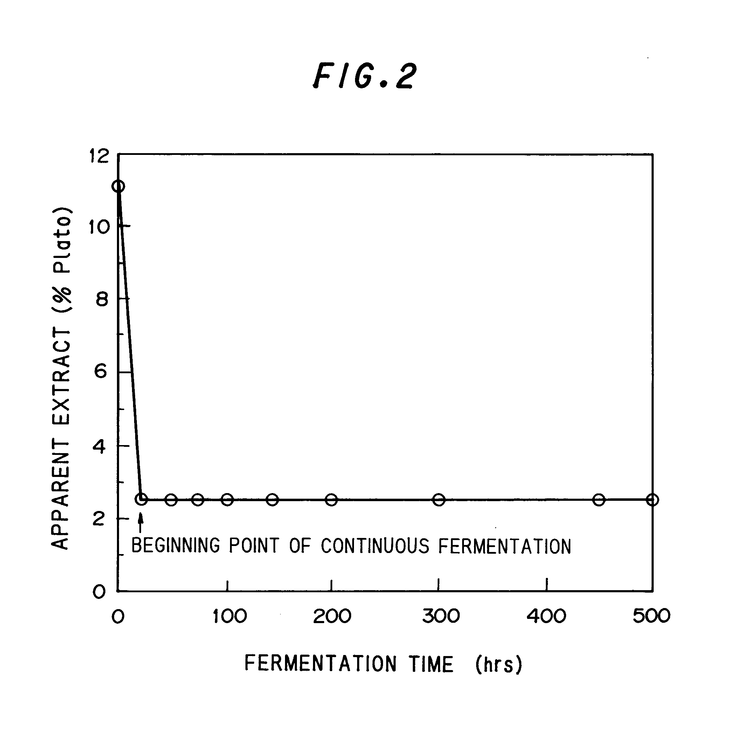

When an apparent extract of the fermentation liquid in the reactor reached 2.5 % Plato (after 19 hours from the start of the fermentation), the wort was fed into the reactor from the lower portion of the reactor at a rate of 40 ml/hr. While the same amount of the fermentation liquid was withdrawn from the upper portion of the reactor, the continuous operation was began in the state of the liquid circulation manner. The results are shown in Fig. 2.

As is apparent from Fig. 2, the apparent extract during the fermentation period was 2.5 % Plato and stable. Further, the concentrations of amino acids in the fermentation liquid were measured as shown in Table 1.

According to the process of the present invention, the amino acids were taken by the yeast and assimilated therein. Therefore, the concentrations of the amino acids in the fermentation liquid were low in comparison to these in the method of Comparative Example and Control.

Table 1

| Comparison in concentrations of amino acids in the fermentation liquid | | | |
|---|---|---|---|
| Amino acid | Concentrations of amino acids (mg/l) | | |
| | Example | Comparative Example | Control |
| Aspartic acid | 3 | 11 | 8 |
| Threonine | 1 | 4 | 1 |
| Serine | 1 | 5 | 2 |
| Glutamic acid | 6 | 34 | 20 |
| Proline | 316 | 341 | 350 |
| Glycine | 14 | 22 | 18 |
| Alanine | 40 | 102 | 60 |
| Valine | 32 | 78 | 32 |
| Methionine | 1 | 9 | 10 |
| Isoleucine | 9 | 20 | 20 |
| Leucine | 11 | 49 | 14 |
| Tyrosine | 43 | 56 | 45 |
| Phenylalanine | 41 | 73 | 40 |
| Lysine | 1 | 25 | 23 |
| Histidine | 12 | 18 | 15 |
| Arginine | 26 | 55 | 52 |

Comparative Example 1

A 500-milliliter packed bed-type reactor was used and packed with 160 ml of chitosan beads having beer yeast [Saccharomyces cerevisiae IAM 4206 (ATCC 9080)] immobilized thereon, and the fermentation was conducted at 11 °C to obtain a fermentation liquid in which an apparent extract was 2.5 % Plato.

In this case, carbon dioxide gas formed during the fermentation was accumulated in the reactor to make a channeling flow of the liquid. As shown in Table 1, since the assimilation of the amino acids by the yeast was unsatisfactory, the concentrations of the amino acids in the fermentation liquid were high. By the way, the concentrations of the amino acids in the fermentation liquid (apparent extract 2.5 % Plato) according to an ordinary method using free yeast cells which were not immobilized are shown in Table 1 as Control.

Example 2

A 1-liter fluidized bed-type reactor was packed with 250 ml of a support material (chitosan beads) having wine yeast [Saccharomyces cerevisiae OC-2 (IAM 4175)] immobilized thereon, and 750 ml of a wort having a sugar degree of 22 % Plato were introduced in the reactor. The batchwise fermentation operation was conducted at 20 °C upon setting a circulation rate of the fermentation liquid in the reactor at 250 ml/min.

When an apparent extract of the fermentation liquid in the reactor reached 4.0 % Plato (after 40 hours from the start of the fermentation). a grape juice was fed into the reactor from the lower portion of the reactor at a rate of 18 ml/hr. While the same amount of the fermentation liquid was withdrawn from the upper portion of the reactor, the continuous operation was begun in the state of the liquid circulation manner.

As a result, the fermentation was conducted stably over 500 hours, and wine could be brewed.

Possibility of Industrial Utilization

According to the present invention, in producing a liquor, a fluidized bed-type reactor loaded with immobilized yeast is used, and a fermentation liquid in the reactor is circulated to cause the immobilized yeast to fluidize, Consequently, most of carbon dioxide gas formed can be exhausted outside the system without being dissolved in the liquid.

Besides, since amino acids in the raw material are assimilated by the yeast at good efficiency, the concentrations of the amino acids in the liquor product are reduced in comparison to those of a conventional method, which gives a good flavor.

**Claims**

**1.** A process for producing a liquor which comprises loading immobilized yeast cells into a fluidized bed-type reactor fitted with a fluid circulation pipe and a gas exhaust port, feeding a raw material for brewing to the reactor, withdrawing part of the culture liquid from the upper portion of the reactor, and returning the culture liquid to the inside of the reactor from the lower portion of the reactor to thereby conduct the cultivation while making a liquid circulation.

**2.** The process of claim 1 wherein during the cultivation, the raw material is fed to the reactor, and part of the culture liquid is withdrawn outside the reactor to produce the liquor continuously.

**3.** The process of claim 1 wherein the reactor is a reactor fitted with a pH sensor and a temperature sensor.

**4.** The process of claim 1 wherein the immobilized yeast cells are liquor producing yeast supported on a support material selected from chitosan, alginic acid and carrageenan.

# FIG.1

EP 0 669 393 A1

# FIG.2

BEGINNING POINT OF CONTINUOUS FERMENTATION

| | International application No. |
|---|---|
| | PCT/JP94/01476 |

**A. CLASSIFICATION OF SUBJECT MATTER**

Int. $Cl^6$   C12C11/09, C12G1/073, C12G3/02

According to International Patent Classification (IPC) or to both national classification and IPC

**B. FIELDS SEARCHED**

Minimum documentation searched (classification system followed by classification symbols)

Int. $Cl^5$   C12C11/00, C12G1/00, C12G3/02

Documentation searched other than minimum documentation to the extent that such documents are included in the fields searched

Electronic data base consulted during the international search (name of data base and, where practicable, search terms used)

WPI

**C. DOCUMENTS CONSIDERED TO BE RELEVANT**

| Category* | Citation of document, with indication, where appropriate, of the relevant passages | Relevant to claim No. |
|---|---|---|
| A | JP, A, 5-317023 (Kikusui Shuzo K.K.), December 3, 1993 (03. 12. 93), (Family: none) | 1-4 |
| A | JP, A, 1-277481 (Le. Ell Liquid S.A. Pull le expuluwatetion de Purocede Jeoruje Claude), November 7, 1989 (07. 11. 89) & EP, A, 334728 | 1-4 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| November 1, 1994 (01. 11. 94) | November 22, 1994 (22. 11. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)